# EUROPEAN PATENT APPLICATION

(11) **EP 0 563 854 A1**
(43) Date of publication of application: **06.10.1993**
(21) Application number: 93105174.2
(22) Date of filing: 29.03.1993
(51) Int. Cl.: A61M 1/02

(54) **Multi-function drainage and reinfusion system for surgical applications**

(30) Priority: 02.04.1992 IT MI920320
(71) Applicant: LUCOMED S.r.L., I-41012 Carpi (Modena) (IT)
(72) Inventor: Bianchi Maiocchi, Costantino, I-20124 Milano (IT)
(74) Representative: Lunati, Vittoriano

(57) **Abstract**

A multi-function drainage and reinfusion system for surgical applications comprises a rigid and air-tight vessel (2) provided with a first base (2a) and a second base (2b) at mutually opposite positions, drawing means comprising a first attachment device (7) fixedly engaged to said first base (2a) and projecting therefrom, a filtering element (8) connected in-line with said drawing means (3), suction means (10) comprising a second attachment device (11) fixedly engaged to said rigid vessel (2) and projecting therefrom, and delivery means (4) comprising a third attachment device (14) fixedly engaged to said second base (2b) and projecting therefrom.

## Description

The present invention relates to a multi-function drainage and reinfusion system for surgical applications.

It is known that following to surgical operations there is the formation in the tissues of hematomas that can constitute a dangerous seat for pathogenic microorganisms, above all in those cases in which the natural absorption of them takes place slowly.

Presently the drainage of such extravasated blood is carried out mainly by appropriate suction devices provided with rubber cannulas inserted in the tissues and opening into collection vessels.

If the amount of blood lost during a surgical operation and in the immediate post-operative course is important, blood transfusions are required.

As it is well known, this therapeutical technique cannot be always put into practice because blood availability is sometimes limited and at all events the risk of transmitting dangerous viruses is always implied in blood transfusions.

For the above reasons, when it is possible, blood drained by the suction devices is purified directly in the operating block through expensive apparatus or sent to laboratories equipped with appropriate purification appliances.

The purified blood is then reinfused to the same patient from whom it had been drawn.

However, the technique of reinfusing his own blood to a patient, as briefly described above, has some drawbacks. In fact, all blood drawn is carried to a laboratory from where it is recovered after a lapse of time which is obviously variable but at all events generally of non negligible importance. Therefore the purified blood is not available at once for reinfusion.

In addition, blood transferring and purification involves the employment of an appropriate staff; thus costs are high and sometimes they may lead not to utilize the reinfusion technique.

In this situation, the object of the present invention is to devise a multi-function drainage and reinfusion system capable of obviating the above drawbacks and enabling the almost immediate reinfusion of the drawn blood at reduced costs, while at the same time ensuring optimal operating conditions and the absence of infection by bacterial microorganisms that may be present in the surrounding atmosphere.

The above specified object is achieved by a multi-function drainage and reinfusion system for surgical applications in accordance with Claim 1.

A description of a system in accordance with the invention is given hereinafter by way of non-limiting example, with reference to the accompanying drawings, in which:
**Figure 1** is a perspective view of the system of the invention taken as a whole;
**Figure 2** is a part exploded side view of the system, in which the vessel is sectioned; and
**Figure 3** is a part cut away view of the system in its use position.

Referring to the drawings, the system in accordance with the invention has been generally identified by reference numeral **1.**

It comprises a rigid vessel **2** substantially of cylindrical form, consisting of two cap-shaped half-shells sealed together at a collar **2c** located substantially in the middle.

The whole vessel 2 is air-tight and is rigid enough to stand the outer atmospheric pressure without undergoing deformation, when the vacuum is created inside it.

Engaged to the rigid vessel 2, through respective attachment devices, is drawing means **3** adapted to send blood to the rigid vessel 2 itself, as well as suction means **10** designed to create a vacuum in the rigid vessel 2, and delivery means **4** adapted to make the blood collected therein available for reinfusion.

The drawing means 3 comprises a first attachment device **7** and a conveying cannula **5** extending between the first attachment device 7 and an end tailpiece **5a** to be connected to the tissues submitted to drainage by appropriate perforated tubes known per se.

A first check valve **5c** is connected to the conveying cannula 5, at an intermediate position thereof, while close to the first attachment device 7 said cannula 5 exhibits an end portion **5b** adapted to be coupled by a first screw threaded connecting element **6** to the first attachment device 7 itself.

The first attachment device 7 is fixedly engaged to a first base **2a** of the rigid vessel 2, is in communication with the inside of the vessel itself and is of the same shape as the cannula 5. In fact it comprises a short length of flexibile hose or first cannula-like element **7a** to which an irreversible-closing element **9** is mounted which is adapted to clamp and completely tighten the first cannula-like element itself 7a.

Also arranged on the multi-function system 1 there is a filtering element **8** provided with a gauze **8a** embodying a "filtering tubular diaphragm for clots", known per se, designed to retain blood clots, fragments of organic tissues and possible other particles present in the extravasated blood submitted to drainage.

The filtering element 8 is connected in-line with the conveying cannula 5 and inserted between the end tailpiece **5a** and the first check valve 5c.

The suction means 10, adapted to produce the vacuum in the rigid vessel 2, specifically comprises a second attachment device **11**, a suction cannula **19**, and a pump.

The second attachment device 11 has a flexibile second cannula-like element **11a**, fixedly engaged to the first base 2a and is in communication with the inside of the rigid vessel 2.

The suction cannula 19 is in the extension of the second cannula-like element 11a and terminates adjacent said second element with a second screw threaded connecting element **21**, while at the opposite end thereof it is connected to a pump. The pump is of the manual type **20** or is embodied by another similar device.

A second check valve **19a** is connected to the suction cannula 19, at an intermediate position thereof; it performs the function of maintaining the vacuum created within the rigid vessel 2.

The delivery means 4 comprises a third attachment device **14** fixedly engagecd to a second base **2b** of the rigid vessel 2 disposed opposite the first base 2a and designed to be directed downwardly when the system 1 is in operation.

In fact a hooking element **15** is provided at the first base 2a for hanging the rigid vessel 2 to a support **15a**.

The third attachment device 14 comprises an outlet **16** and a third cannula-like element **14a** provided at one end thereof with a pierceable-diaphragm connection attachment **17** for engagement with pretransfusion filters known per se.

A housing **18** is provided outwardly of the second base 2b of the rigid vessel 2, into which the pierceable-diaphragm connection attachment 17 can be forcedly fitted when the delivery means 4 is not used, in order to prevent the third cannula-like element 14a from dangling.

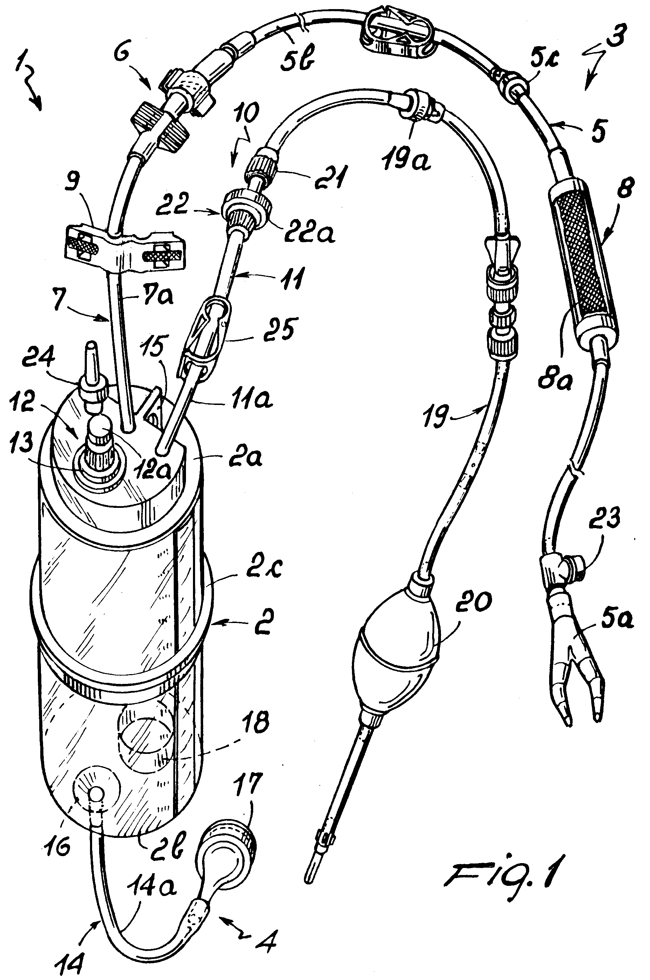

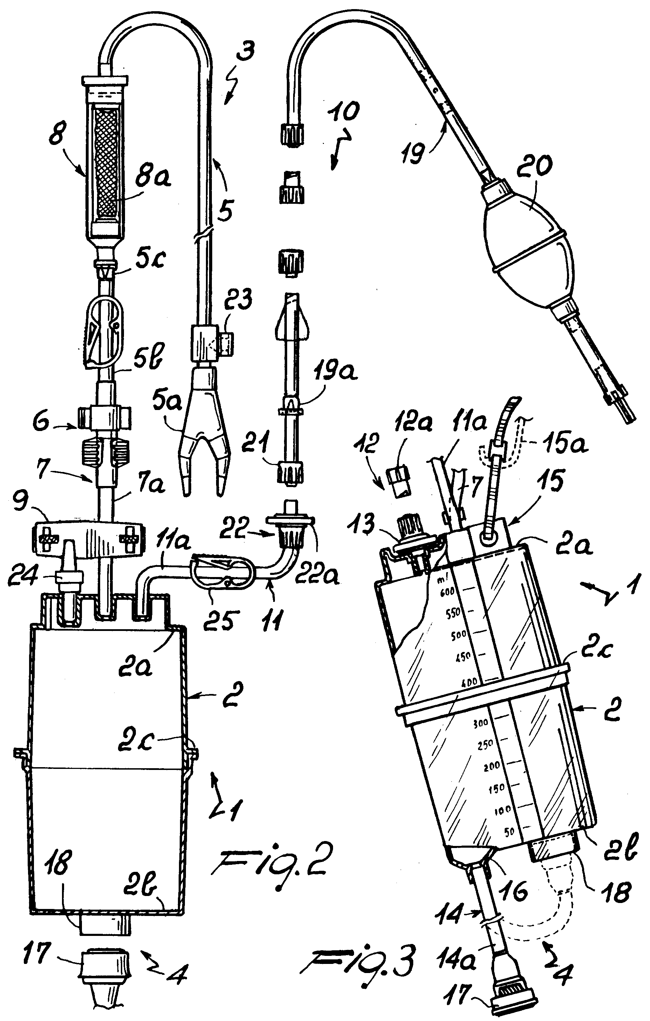

As shown in Figure 3, the hooking element 15 and outlet 16 are located close to opposite generating lines of the rigid vessel 2 so that the outlet 16 will be disposed at the lowermost position of the second base 2b in order to ensure the complete delivery of all the collected blood.

Provision is also made for control means comprising air admission valves and infusion points adapted to adjust the blood flow and enable intervention of an operator whenever necessary.

In more detail, a first air admission valve **12** is arranged on the first base 2a of the rigid vessel 2, which valve is provided with a sealing stopper **12a** and a first hydrophobic antibacterial air filter **13**, known per se, at the inside thereof.

In addition, a second air admission valve **22** provided at the inside thereof with a second hydrophobic antibacterial air filter **22a** is disposed at the end of the second attachment device 11.

Then there is a snap-fit closing element **25** between the second air admission valve 22 and the rigid vessel 2, which element 25 may be selectively closed or opened and is adapted to clamp the second attachment device 11 when the second screw threaded connecting element 21 is unscrewed.

As viewed from Figures 1 and 2, immediately close to the end tailpiece 5a of the conveying cannula 5 there is a first infusion point **23** for anticoagulant substances and a second infusion point **24** for anticoagulant substances too, is disposed on the first base 2a of the rigid vessel 2.

The whole rigid vessel 2 is made of transparent plastic material and after welding at the collar 2c it appears unitary and without movable parts.

In addition the rigid vessel 2 is fixedly joined to the attachment devices 7, 11, 14, the first air admission valve 12 and the second infusion point 24.

In the connection it is pointed out that the first and second base 2a, 2b of the rigid vessel 2 have a shape matching the shape of the above members, as shown in Figures 2 and 3, so that an optimal engagement between them can be achieved. In particular the first base 2a is expanded so that it can accommodate deep housings for engagement with the elements fixedly connected thereto.

In addition the hooking element 15 and housing 18 are formed in the bases 2a, 2b themselves.

The system is sterilized by radiation (Gamma beams) so that also the air initially present therein is sterilized.

Operation of the system is as follows.

In the draining step, blood filtered by the filtering element 8 is conveyed by the conveying cannula 5 into the rigid vessel 2 where in a manual pump 20 driven now and then, creates the vacuum to a degree sufficient to enable blood suction.

Anticoagulant substances are added through the first infusion point 23.

Meanwhile the first air admission valve 12 is maintained closed by the stopper 12a.

When filling of the rigid vessel 2 is completed, the irreversible-closing element 9 is activated so that it hermetically tightens the first attachment device 7 and, after unscrewing the first screw threaded connection element 6, the conveying cannula 5 can be removed and if necessary connected to another rigid vessel for going on drainage.

The reinfusion step can begin immediately after connecting a pretransfusion filter provided with a defluxion member known per se to the pierceable-diaphragm connection attachment 17 picked up from the housing 18.

For blood delivery it is necessary to take away the stopper 12a of the air admission valve 12 and bring the inside of the rigid vessel 2 back to the atmospheric pressure.

The first hydrophobic antibacterial air filter 13 present in the first air admission valve 12 keeps the air flow towards the inside of the vessel in a sterile state and prevents possible blood leakages.

As can be viewed from Figure 3, the position of the outlet 16 enables the rigid vessel 2 to be completely emptied.

If it is necessary to carry out a quicker outflow of the blood it is possible to unscrew the second connecting element 21, thereby bringing also the second air admission valve 22 provided with the second hydrophobic antibacterial air filter 22a into direct communication with the surrounding atmosphere.

Said second filter acts in the same manner as the first filter 13.

By virtue of the snap-fit closing element 25, the second air admission valve 22 may be substantially deactivated even when it is in direct communication with the atmopshere.

The invention attains important advantages.

In fact, the presence of the hydrophobic antibacterial air filters 13 and 22a ensures not only that contaminations with the surrounding atmosphere do not occur, but also that blood does not come out of the rigid vessel 2 even if the latter is accidentally dropped or submitted to shakings.

The downward positioning of the third attachment device 14 at all events prevents any manipulation of the vessel during the reinfusion step.

In addition, all operations to be carried out are performed while the rigid vessel 2 is kept closed and said operations are mainly executed on the attachment devices 7, 11, 14.

The latter, being flexible and projecting to a great extent from the rigid vessel, represent engagement seats for the elements to be connected and also enable said elements to be mounted thereto without touching or moving the rigid vessel 2.

Thus the operations to be executed at the attachment devices are simpler and safer.

It is pointed out that the first and second screw threaded connecting elements 6 and 21, and the pierceable-diaphragm connection attachment 17 ensure the greatest versatility to the system of the invention which can be utilized in different ways and connected to the appropriate elements without altering its fundamental structure.

The system enables drainage, filtering and reinfusion functions to be carried out in an excellent manner.

In fact, the thorough drainage is ensured by the hermetic seal of the rigid vessel 2 that can be easily made and connected to the other elements so as to prevent air penetration when the vacuum is created therein.

All sealed points in fact are not critical and can be made by adopting means known per se for joining a rigid element (the rigid vessel 2) to other rigid elements (the first air admission valve 12 and second infusion point 24) or to elements of at least some thickness and almost rigid over short lengths (the cannula-like elements 7a, 11a, 14a).

The rigid vessel itself 2 consists of rigid half-shells perfectly sealed with each other at the collar 2c and has bases 2a, 2b the shape of which matches that of said cannula-like elements, so as to ensure a steady union therewith.

Under these sealing conditions, the vacuum created by few initial squeezings of the manual pomp 20 is sufficient to enable suction and lasts permanently.

Therefore the permanent presence of qualified staff is not required with this system.

Also the first infusion point 23 for introducing an anticoagulant substance and placed close to the end tailpiece 5a promotes flowing of the drained blood.

An optimal filtration is ensured by the filtering element 8. This element may be arranged to have the most appropriate sizes and also in series with other similar elements, being spaced apart from the rigid vessel 2.

An excellent reinfusion is ensured both by the second infusion point 24 enabling blood to be fluidified with anticoagulant substances directly introduced into the rigid vessel 2, and by the possibility of admitting filtered air to the rigid vessel 2 in order to eliminate the vacuum and compensate for progressive emptying, through one or two (at choice) air admission valves provided with respective hydrophobic antibacterial air filters 13 and 22a.

Air admission can therefore be effected at a sufficient speed, in spite of the high filtering capabilities of the hydrophobic antibacterial air filters 13 and 22a.

Thus the invention complies with the opposing requirements of providing antibacterial air filters as much as possible fine and therefore greatly hindering the air flow, and carrying out a quick compensation for the vacuum created during reinfusion due to the stored blood going out.

## Claims

1. A multi-function drainage and reinfusion system for surgical applications, characterized in that it comprises:
- a rigid air-tight vessel (2) provided with a first base (2a) and a second base (2b) at mutually opposite positions,
- drawing means (3) adapted to convey blood into said rigid vessel (2) and comprising a first attachment device (7) fixedly engaged to said first base (2a) and projecting therefrom,
- at least one filtering element (8) spaced apart from said rigid vessel (2) and connected with said drawing means (3),
- suction means (10) adapted to extract air from said rigid vessel (2) and comprising a second attachment device (11) fixedly engaged to said rigid vessel (2) and projecting therefrom, and
- delivery means (4) designed to reinfuse the filtered blood collected in said rigid vessel (2) and comprising a third attachment device (14) fixedly engaged to said second base (2b) and projecting therefrom.

2. A system according to Claim 1, wherein control means designed to enable adjustment of the blood flow is arranged, which means comprises at least a first and a second air admission valves (12, 22) internally provided with a first and a second hydrophobic antibacterial air filters (13, 22a), said first and second air admission valves (12, 22) being in communication with said rigid vessel (2) and being able to be opened and closed independently of each other for adjusting the air flow in said rigid vessel (2).

3. A system according to Claim 2, wherein said control means comprises a first infusion point (23) for admission of anticoagulant substances located on said drawing means (3) and a second infusion point (24) located on said rigid vessel (2).

4. A system according to Claim 1, wherein said drawing means (3) comprises a conveying cannula (5) disposed in the extension of said attachment device (7) and detachably engaged to the device itself through a first screw threaded connecting element (6), said filtering element (8) being placed along said conveying cannula (5).

5. A system according to Claim 1, wherein said delivery means (4) comprises a pierceable-diaphragm connection attachment (17) placed at the end of said third attachment device (14), and a housing (18) for said pierceable-diaphragm connection attachment (17) located externally of said rigid vessel (2) and being part of the same.

6. A system according to Claim 1, wherein said suction means (10) comprises a suction cannula (19) located in the extension of said second attachment device (11) and detachably engaged to the device itself through a second screw threaded connecting element (21), said suction cannula (19) being connected at its other end to a pump (20).

7. A system according to Claims 2 and 6, wherein said second air admission valve (22) is fixedly engaged to said second attachment device (11) and is closed to said second screw threaded connection element (21).

8. A system according to Claim 2, wherein said first air admission valve (12) is fixedly engaged to said first base (2a).

9. A system according to Claim 1, wherein said first, second and third attachment devices (7, 11, 14) comprise a first, second and third cannula-like element (7a, 11a, 14a) respectively, which elements are flexible, project from said first and second base (2a, 2b) and are fixedly engaged to said first and second base (2a, 2b).

10. A system according to Claim 9, wherein said first and second base (2a, 2b) have shapes matching ends of said first, second and third cannula-like elements (7a, 11a, 14a).

11. A system according to Claim 1, wherein said rigid vessel (2) is substantially cylindrical and it is formed from two half-shells sealingly welded together at a collar (2c) located substantially intermediate said first and second base (2a, 2b).

12. A system according to Claim 1, wherein said rigid vessel (2) is provided, at said first base (2a), with a hooking element (15), said hooking element (15) and said third attachment device (14) projecting from said rigid vessel (2) close to opposite generating lines of said rigid vessel (2).
